# FASCICULE DE BREVET EUROPEEN

(11) **EP 0 613 682 B1**
(45) Date de publication et mention de la délivrance du brevet: **24.07.1996**
(21) Numéro de dépôt: 94400207.0
(22) Date de dépôt: 01.02.1994
(51) Int. Cl.: A61K 7/48, A61K 7/00

(54) **Composition cosmétique ou dermopharmaceutique constituée d'une émulsion contenant au moins une gomme de silicone et son utilisation**
Kosmetische dermopharmazeutische Zusammensetzung, bestehend aus einer Emulsion, die mindestens ein Silikon-Gummi enthält und Verwendung
Cosmetic or dermopharmaceutic composition constituted by an emulsion containing at least a silicone-gum and its use

(30) Priorité: 26.02.1993 FR 9302211
(43) Date de publication de la demande: 07.09.1994
(73) Titulaire: L'OREAL, F-75008 Paris (FR)
(72) Inventeur: Afriat, Isabelle, F-75014 Paris (FR); Guth, Gérard, F-95160 Montmorency (FR); Fodor, Pierre, F-92380 Garches (FR)
(74) Mandataire: Peuscet, Jacques

(56) Documents cités:
- EP-A- 0 290 166
- EP-A- 0 424 260
- EP-A- 0 492 657
- WO-A-92/09263
- FR-A- 2 679 769

## Description

La présente invention concerne une composition cosmétique ou dermopharmaceutique constituée d'une émulsion stable de type huile dans l'eau (ci-après désignée par "émulsion H/E") contenant dans sa phase huileuse au moins une gomme de silicone l'invention concerne également l'utilisation d'une telle composition pour les soins de la peau et comme produit de maquillage.

Les produits de soins ou de maquillage de la peau sont souvent des émulsions d'une phase huileuse ou phase grasse dans une phase aqueuse ; il faut évidemment qu'une telle émulsion soit stable au cours du temps pour être acceptable sur le plan commercial. Généralement, on assure la stabilité desdites émulsions en y incorporant des agents tensioactifs comme émulsionnants. Malheureusement, la présence d'agents tensioactifs est souvent une source d'inconfort ou d'irritation de la peau et il est souhaitable de l'éviter.

Les gommes de silicone constituent des ingrédients particulièrement recherchés en cosmétique car elles permettent de conférer à la peau d'excellentes propriétés de lubrification et de résistance à l'eau ; par ailleurs, elles forment sur la peau un film protecteur, qui la protège de la déshydratation, sans que l'on observe un effet gras.

Les gommes de silicone sont avantageuses par rapport aux autres dérivés de silicone, en particulier par rapport aux huiles de silicone, qui sont des polydiméthylsiloxanes ou des polydiphénylsiloxanes de faible viscosité. En effet, elles apportent plus de douceur à la peau et des propriétés filmogènes supérieures. Cependant, jusqu'à présent, il n'a pas été possible d'incorporer de façon satisfaisante des proportions appréciables de gomme de silicone dans des émulsions H/E, en l'absence de tensioactif. Au demeurant, avec les gommes de silicone, même la présence de tensioactifs ne résolvait pas le problème de la stabilité : il se produisait généralement, dès que le pourcentage de gomme de silicone était élevé, une coalescence de la gomme de silicone et on obtenait des émulsions grossières et peu stables, impropres à l'utilisation en cosmétique.

On a déjà proposé dans la demande de brevet JP-02-019310, une composition de maquillage constituée par une dispersion huile dans l'eau, dont la stabilité est assurée grâce à l'incorporation, dans la phase aqueuse, de polymères acryliques réticulés. De même, dans EP-A-268 164 et EP-A 332 501, on a décrit la stabilisation d'une émulsion H/E à l'aide de polymères acryliques. Dans la phase grasse de ces compositions, on ne trouve néanmoins que des huiles et jamais de gomme de silicone ; et il a été vérifié, en effet, que les gommes de silicone ne sont pas stabilisées par les systèmes décrits.

Selon la présente invention, on a trouvé que l'on pouvait obtenir des émulsions H/E stables contenant dans leur phase huileuse une gomme de silicone, sans qu'il soit nécessaire d'ajouter un agent tensioactif émulsionnant, pour assurer leur stabilité en introduisant dans la phase aqueuse de l'émulsion un agent épaississant constitué par un copolymère acrylique particulier.

La présente invention a donc pour objet une composition cosmétique ou dermopharmaceutique constituée d'une émulsion de type huile dans l'eau, stable en l'absence d'agent tensioactif, caractérisée par le fait que la phase huileuse contient au moins une gomme de silicone et la phase aqueuse contient au moins un copolymère d'acide acrylique et d'au moins un monomère vinylique portant au moins une fonction prise dans le groupe formé par les fonctions amide, amidine et nitrile.

Le copolymère d'acide acrylique et d'au moins un monomère vinylique contenu dans la phase aqueuse des compositions selon l'invention, telles que ci-dessus défini, est ci-après désigné sous le nom "copolymère (C)". Le copolymère (C) est, de préférence, celui identifié dans le dictionnaire CTFA sous la dénomination "acrylic acid/acrylonitrogen copolymer", à savoir un copolymère d'acide acrylique et de monomères vinyliques portant des fonctions amide, amidine et nitrile obtenu par hydrolyse contrôlée de polyacrylonitrile. C'est plus particulièrement le copolymère vendu sous la dénomination commerciale "HYPAN SA 100H" par la société "LIPO CHEMICALS INC".

Le copolymère (C) est, de préférence, au moins partiellement neutralisé à l'aide d'agent(s) alcalin(s) classique(s) pour faciliter son gonflement dans l'eau. De façon préférentielle, on utilise pour la neutralisation au moins une alcanolamine telle que la N,N,N',N'-tétrakis(2-hydroxypropyl)éthylène diamine, la triéthanolamine, le tris(hydroxyméthyl)aminométhane ou le 2-amino 2-méthyl 1-propanol.

Les gommes de silicone utilisées selon la présente invention, sont des polysiloxanes ayant un poids moléculaire élevé pouvant aller, par exemple, de 200 000 à 1 000 000.

Les gommes utilisables selon l'invention sont par exemple des polyalkylsiloxane ayant des groupes terminaux triméthylsilyle dénommés "diméthicone" selon la nomenclature du dictionnaire CTFA, des polyalkylsiloxanes ayant des groupes terminaux trihydroxysilyle dénommés "diméthiconol" selon la nomenclature du dictionnaire CTFA, ou les copolymères suivants : poly(diméthylsiloxane/méthylvinylsiloxane), poly(diméthylsiloxane/diphénylsiloxane), poly(diméthylsiloxane/phénylméthylsiloxane), poly(diméthylsiloxane/diphénylsiloxane/méthylvinylsiloxane). Ces gommes peuvent être utilisées telles quelles ou, de préférence, en mélange ou en solution dans une huile de silicone telle qu'une huile de polydiméthylsiloxane ou une huile de polyphénylméthylsiloxane. Les gommes de silicone ou les mélanges de gomme et d'huile de silicone peuvent être utilisés en solution dans un solvant tel que les isoparaffines, le chlorure de méthylène, le pentane, le dodécane, le tridécane, le tétradécane ou leurs mélanges.

Des produits plus particulièrement utilisables sont les mélanges de gomme et d'huile de silicone tels que :
- les mélanges de diméthiconol et d'un polydiméthylsiloxane cyclique ; un exemple d'un tel mélange est le produit vendu sous la dénomination "Q2-1401" par la société "DOW CORNING" ;
- les mélanges de diméthiconol et d'un polydiméthylsiloxane ayant une viscosité de 5.10⁻⁴ m²/s ; un exemple d'un tel mélange est le produit vendu sous la dénomination "Q2-1403" par la société "DOW CORNING" ;
- les mélanges formés à partir d'une gomme de polydiméthylsiloxane avec une huile de silicone cyclique ; un exemple d'un tel mélange est le produit vendu sous la dénomination "SF1214 Silicone Fluid" par la société "GENERAL ELECTRIC" qui est un mélange d'une diméthicone ayant un poids moléculaire de 500 000 et de décaméthylcyclopentasiloxane ;
- les mélanges d'une gomme de polydiméthylsiloxane et d'une huile de polydiméthylsiloxane ; des exemples d'un tel mélange sont les produits commercialisés sous les dénominations "SF1236" et "CF1241" par la société "GENERAL ELECTRIC" ; le produit "SF1236" est un mélange de 15 % d'une gomme de polydiméthylsiloxane ayant un poids moléculaire de 500 000 et une viscosité de 20 m2/s dénommée "SE30" et de 85 % d'une huile de polydiméthylsiloxane ayant une viscosité de 5.10⁻⁶ m²/s ; le produit "CF1241" est un mélange de 33 % de gomme "SE30" et de 67 % d'un polydiméthylsiloxane ayant une viscosité de 10⁻³ m²/s.

Selon l'invention, la phase aqueuse de l'émulsion représente, de préférence, 45 à 90 % en poids par rapport au poids total de la composition et la phase huileuse représente le complément c'est-à-dire, de préférence, de 10 à 55 % en poids par rapport au poids total de la composition. Le copolymère (C) est présent, dans la phase aqueuse, en une proportion variant entre 0,05 et 1 % en poids, de préférence, entre 0,1 et 0,5 % en poids par rapport au poids total de la composition.

La quantité de gomme de silicone présente dans la phase huileuse de l'émulsion est supérieure à 8 %, de préférence, comprise entre 10 et 80 % en poids par rapport au poids total de la phase huileuse de l'émulsion.

La phase huileuse peut contenir avantageusement, en plus de la (des) gomme(s) de silicone ou du mélange de gomme(s) de silicone et d'huile de silicone, au moins une huile végétale, animale, minérale ou synthétique. Parmi les huiles végétales, on peut citer l'huile de jojoba, l'huile d'olive, l'huile d'amande douce, l'huile d'avocat, l'huile de coco, l'huile de germe de blé, l'huile de maïs, l'huile de palme, l'huile de macadamia, l'huile de sésame, l'huile de soja, l'huile d'argan, l'huile d'onagre, l'huile de bourrache et les huiles essentielles. Parmi les huiles animales, on peut notamment citer l'huile de poisson. Parmi les huiles minérales, on peut citer l'huile de vaseline et les isoparaffines. Parmi les huiles synthétiques, on peut mentionner les palmitates d'éthyle et d'isopropyle, les palmitates d'octyle et d'éthyl-2 hexyle, les myristates d'alkyle tels que le myristate d'isopropyle, de butyle et de cétyle, le stéarate d'hexyle, les triglycérides des acides octanoïque et décanoïque, le ricinoléate de cétyle, l'octanoate de stéaryle, le monococoate d'éthylhexyle et le stéarate de glycéryle.

Selon l'invention, la phase aqueuse peut contenir, en plus du copolymère (C), un second polymère acrylique hydrosoluble qui est un homo- ou copolymère d'acide acrylique, éventuellement réticulé, ou un de ses sels. Le second polymère est, par exemple, un de ceux commercialisés sous la dénomination "CARBOPOL" par la société "GOODRICH". Ce second polymère est présent dans des proportions comprises entre 0,1 et 2 % et, de préférence, entre 0,2 et 1 % en poids par rapport au poids total de l'émulsion. Ce second polymère confère aux émulsions de meilleures qualités cosmétiques.

L'émulsion, qui constitue la composition selon l'invention, est avantageusement préparée par dispersion du copolymère (C) dans de l'eau contenant une quantité suffisante d'un agent neutralisant pour neutraliser au moins partiellement le copolymère (C) et permettre le gonflement du copolymère (C), et par addition lente de la phase huileuse dans la phase aqueuse, en agitant fortement. Quand la phase aqueuse contient un polymère acrylique autre que le copolymère (C), celui-ci est ajouté dans la phase aqueuse avant l'agitation, la neutralisation intervenant après la réalisation de l'émulsion, l'agent neutralisant étant de même nature que celui utilisé pour le copolymère (C).

La composition selon l'invention peut contenir, en outre, au moins un additif ou un actif couramment utilisé en cosmétique ou dans le domaine dermopharmaceutique ; ces adjuvants sont soit dans la phase huileuse de l'émulsion s'ils sont liposolubles, soit dans la phase aqueuse s'ils sont hydrosolubles. Parmi ces additifs ou actifs, on peut citer, sans que cette liste soit considérée comme limitative, les colorants, les conservateurs, les parfums, les filtres solaires, les antioxydants, les hydratants tels que la glycérine, les vitamines et les agents antiradicaux libres. La composition peut également contenir des solides pulvérulents tels que des pigments ou des charges, qui sont en suspension dans l'une des deux phases de la composition. Ces solides pulvérulents sont présents en quantité ne dépassant pas 15 % du poids total de la composition, de préférence en quantité comprise entre 0,1 et 10 % du poids total de la composition.

L'invention concerne également une utilisation de la composition ci-dessus définie comme crème ou lait pour les soins de la peau, notamment crème pour le visage ou pour le corps, lait démaquillant ou lait pour le corps. Elle concerne également une utilisation de ladite composition comme produit de maquillage, notamment comme mascara, fond de teint, eye-liner, fard à joues ou fard à paupières.

Les exemples donnés ci-après, à titre illustratif et nullement limitatif, permettront de mieux comprendre l'invention.

### EXEMPLE 1 : Crème de soin pour le visage

On prépare :
A) Une phase aqueuse ayant la composition suivante :

| | |
|---|---|
| - Copolymère d'acide acrylique et de monomères vinyliques vendu sous la dénomination commerciale "HYPAN SA 100H" par la société "LIPO CHEMICALS INC" | 0,30 g |
| - N,N,N',N'-tétrakis(2-hydroxypropyl) éthylène diamine vendue sous la dénomination commerciale "QUADROL L" par la société "BASF" | 0,35 g |
| - Eau | 49,35 g |

B) Une phase huileuse ayant la composition suivante :

| | |
|---|---|
| - Gomme de silicone vendue sous la dénomination commerciale "Q2 1403" par la société "DOW CORNING" | 50 g |

On mélange les deux phases sous agitation énergique. On obtient une crème blanche, apportant de la douceur et résistant à l'eau. On a constaté que cette crème était restée stable après 3 mois de stockage.

### EXEMPLE 2 : Crème de soin pour le visage.

On a mélangé les phases suivantes :
A) Phase aqueuse

| | |
|---|---|
| - Copolymère d'acide acrylique et de monomères vinyliques vendu sous la dénomination commerciale "HYPAN SA 100H" par la société "LIPO CHEMICALS INC" | 0,30 g |
| - N,N,N',N'-tétrakis(2-hydroxypropyl) éthylène diamine vendue sous la dénomination commerciale "QUADROL L" par la société "BASF" | 0,40 g |
| - Acide polyacrylique réticulé vendu sous la dénomination commerciale "CARBOPOL 940" par la société "GOODRICH" | 0,30 g |
| - Triéthanolamine | 0,30 g |
| - Eau | 88,70 g |

B) Phase huileuse :

| | |
|---|---|
| - Gomme de silicone vendue sous la dénomination commerciale "Q2 1403" par la société "DOW CORNING" | 10 g |

On obtient une crème blanche, s'étalant aisément sur la peau et lui conférant beaucoup de douceur. On a constaté que cette crème était restée stable après 3 mois de stockage.

### EXEMPLE 3 : Crème de soin pour le visage.

On a mélangé les phases suivantes :
A) Phase aqueuse

| | |
|---|---|
| - Copolymère d'acide acrylique et de monomères vinyliques vendu sous la dénomination commerciale "HYPAN SA 100H" par la société "LIPO CHEMICALS INC" | 0,30 g |
| - N,N,N',N'-tétrakis(2-hydroxypropyl) éthylène diamine vendue sous la dénomination commerciale "QUADROL L" par la société "BASF" | 0,50 g |
| - Eau | 78,55 g |

B) Phase huileuse :

| | |
|---|---|
| - Gomme de silicone vendue sous la dénomination commerciale "Q2 1403" par la société "DOW CORNING" | 3,00 g |
| - Acides gras polyinsaturés | 3,00 g |
| - Palmitate d'octyle | 7,00 g |
| - Vaseline | 2,00 g |
| - Huile de jojoba | 1,50 g |
| - Alcool cétylique | 1,00 g |
| - Stéarate de glycérile non autoémulsionnable | 2,50 g |
| - Tocophérols | 0,20 g |
| - Acide citrique | 0,25 g |
| - Parfum | 0,20 g |

On obtient une crème blanche, hydratante, conférant beaucoup de douceur à la peau. On a constaté que cette crème était restée stable après 3 mois de stockage.

## Revendications

1. Composition cosmétique ou dermopharmaceutique constituée d'une émulsion de type huile dans l'eau, stable en l'absence d'agent tensioactif, caractérisée par le fait que la phase huileuse contient au moins une gomme de silicone et la phase aqueuse contient au moins un copolymère (C) d'acide acrylique et d'au moins un monomère vinylique portant au moins une fonction prise dans le groupe formé par les fonctions amide, amidine et nitrile.

2. Composition selon la revendication 1, caractérisée par le fait que le copolymère (C) est un copolymère d'acide acrylique et de monomères vinyliques portant des fonctions amide, amidine et nitrile.

3. Composition selon l'une des revendications 1 ou 2, caractérisée par le fait que le copolymère (C) est au moins partiellement neutralisé.

4. Composition selon la revendication 3, caractérisée par le fait que le copolymère est neutralisé par au moins une alcanolamine.

5. Composition selon la revendication 4, caractérisée par le fait que l'alcanolamine est choisie dans le groupe formé par la N,N,N',N'-tétrakis(2-hydroxypropyl)éthylène diamine, la triéthanolamine, le tris(hydroxyméthyl)aminométhane et le 2-amino 2-méthyl 1-propanol.

6. Composition selon l'une des revendications 1 à 5, caractérisée par le fait que la gomme de silicone est choisie dans le groupe formé par les polyalkylsiloxanes ayant des groupes terminaux triméthylsilyle ou trihydrosilyle et les copolymères suivants poly(diméthylsiloxane/méthylvinylsiloxane), poly(diméthylsiloxane/diphénylsiloxane), poly(diméthylsiloxane/phénylméthylsiloxane) et poly(diméthylsiloxane/diphénylsiloxane/méthylvinylsiloxane).

7. Composition selon l'une des revendications 1 à 6, caractérisée par le fait que les gommes sont en mélange ou en solution dans une huile de silicone.

8. Composition selon la revendication 7, caractérisée par le fait que l'huile de silicone est une huile de polydiméthylsiloxane ou une huile de polyphénylméthylsiloxane.

9. Composition selon l'une des revendications 1 à 7, caractérisée par le fait que la gomme de silicone ou le mélange de gomme de silicone et d'huile de silicone est en solution dans un solvant.

10. Composition selon la revendication 9, caractérisée par le fait que le solvant est choisi dans le groupe formé par les isoparaffines, le chlorure de méthylène, le pentane, le dodécane, le tridécane, le tétradécane et leurs mélanges.

11. Composition selon l'une des revendications 1 à 10, caractérisée par le fait que la phase aqueuse de l'émulsion représente de 45 à 90 % en poids par rapport au poids total de la composition, la phase huileuse représentant le complément.

12. Composition selon l'une des revendications 1 à 11, caractérisée par le fait que le copolymère (C) est présent dans la phase aqueuse de l'émulsion en une proportion comprise entre 0,05 et 1 % en poids par rapport au poids total de la composition et, de préférence, entre 0,1 et 0,5 % en poids par rapport au poids total de la composition.

13. Composition selon l'une des revendications 1 à 12, caractérisée par le fait que la phase huileuse contient, en plus de la (ou des) gomme(s) de silicone ou du mélange de gomme(s) de silicone et d'huile de silicone, au moins une huile prise dans le groupe formé par les huiles végétales, animales, minérales et synthétiques.

14. Composition selon l'une des revendications 1 à 13, caractérisée par le fait que la phase aqueuse contient en plus du copolymère (C), au moins un homo- ou copolymère d'acide acrylique, réticulé ou non réticulé, ou un de ses sels.

15. Compositon selon la revendication 14, caractérisée par le fait que le polymère d'acide acrylique autre que le copolymère (C) est présent dans la phase aqueuse de l'émulsion en une proportion comprise entre 0,1 et 2 % en poids par rapport au poids total de la composition.

16. Utilisation cosmétique d'une composition selon l'une des revendications 1 à 15 comme crème ou lait pour les soins de la peau.

17. Utilisation d'une composition selon l'une des revendications 1 à 15 pour constituer un produit de maquillage pris dans le groupe formé par les fonds de teint, les mascaras, les fards à joues, les fards à paupières et les eye-liners.

## Claims

1. Cosmetic or dermopharmaceutical composition composed of an emulsion of oil-in-water type which is stable in the absence of surface-active agent, characterized in that the oily phase contains at least one silicone gum and the aqueous phase contains at least one copolymer (C) of acrylic acid and of at least one vinyl monomer carrying at least one functional group taken from the group formed by amide, amidine and nitrile functional groups.

2. Composition according to Claim 1, characterized in that the copolymer (C) is a copolymer of acrylic acid and of vinyl monomers carrying amide, amidine and nitrile functional groups.

3. Composition according to either of Claims 1 and 2, characterized in that the copolymer (C) is at least partially neutralized.

4. Composition according to Claim 3, characterized in that the copolymer is neutralized by at least one alkanolamine.

5. Composition according to Claim 4, characterized in that the alkanolamine is chosen from the group formed by N,N,N',N'-tetrakis(2-hydroxypropyl)ethylenediamine, triethanolamine, tris(hydroxymethyl)aminomethane and 2-amino-2-methyl-1-propanol.

6. Composition according to one of Claims 1 to 5, characterized in that the silicone gum is chosen from the group formed by polyalkylsiloxanes having trimethylsilyl or trihydrosilyl end groups and the following copolymers: poly(dimethylsiloxane/methylvinylsiloxane), poly(dimethylsiloxane/diphenylsiloxane), poly(dimethylsiloxane/phenylmethylsiloxane) and poly(dimethylsiloxane/diphenylsiloxane/methylvinyl-siloxane).

7. Composition according to one of Claims 1 to 6, characterized in that the gums are present as a mixture or in solution in a silicone oil.

8. Composition according to Claim 7, characterized in that the silicone oil is a polydimethylsiloxane oil or a polyphenylmethylsiloxane oil.

9. Composition according to one of Claims 1 to 7, characterized in that the silicone gum or the mixture of silicone gum and of silicone oil is in solution in a solvent.

10. Composition according to Claim 9, characterized in that the solvent is chosen from the group formed by isoparaffins, methylene chloride, pentane, dodecane, tridecane, tetradecane and their mixtures.

11. Composition according to one of Claims 1 to 10, characterized in that the aqueous phase of the emulsion represents from 45 to 90 % by weight with respect to the total weight of the composition, the oily phase representing the remainder.

12. Composition according to one of Claims 1 to 11, characterized in that the copolymer (C) is present in the aqueous phase of the emulsion in a proportion of between 0.05 and 1 % by weight with respect to the total weight of the composition and, preferably, between 0.1 and 0.5 % by weight with respect to the total weight of the composition.

13. Composition according to one of Claims 1 to 12, characterized in that the oily phase contains, in addition to the silicone gum(s) or the mixture of silicone gum(s) and of silicone oil, at least one oil taken from the group formed by vegetable, animal, mineral and synthetic oils.

14. Composition according to one of Claims 1 to 13, characterized in that the aqueous phase contains, in addition to the copolymer (C), at least one homo- or copolymer of acrylic acid, which may or may not be crosslinked, or one of its salts.

15. Composition according to Claim 14, characterized in that the polymer of acrylic acid, other than the copolymer (C), is present in the aqueous phase of the emulsion in a proportion of between 0.1 and 2 % by weight with respect to the total weight of the composition.

16. Cosmetic use of a composition according to one of Claims 1 to 15 as a cream or milk for caring for the skin.

17. Use of a composition according to one of Claims 1 to 15 for constituting a make-up product taken from the group formed by foundations, mascaras, blushers, eye shadows and eyeliners.

## Patentansprüche

1. Kosmetische oder hautpharmazeutische Zusammensetzung, bestehend aus einer bei Fehlen eines Tensids stabilen Öl-in-Wasser-Emulsion, dadurch gekennzeichnet, daß die Ölphase mindestens einen Silikongummi und die wässrige Phase mindestens ein Kopolymer (C) aus Akrylsäure und mindestens einem Vinylmonomer enthält, das mindestens eine funktionelle Gruppe trägt, die aus der von den funktionellen Amid-, Amidin- und Nitrilgruppen gebildeten Gruppe gewählt ist.

2. Zusammensetzung nach Anspruch 1, dadurch gekennzeichnet, daß das Kopolymer (C) ein Kopolymer aus Akrylsäure und Vinylmonomeren mit funktionellen Amid-, Amidin- und Nitrilgruppen ist.

3. Zusammensetzung nach einem der Ansprüche 1 oder 2, dadurch gekennzeichnet, daß das Kopolymer (C) mindestens partiell neutralisiert ist.

4. Zusammensetzung nach Anspruch 3, dadurch gekennzeichnet, daß das Kopolymer durch mindestens ein Alkanolamin neutralisiert ist.

5. Zusammensetzung nach Anspruch 4, dadurch gekennzeichnet, daß das Alkanolamin aus der Gruppe ausgewählt ist, die von N,N,N',N'-Tetrakis(2-hydroxypropyl)ethylendiamin, Triethanolamin, Tris(hydroxymethyl)aminomethan und 2-Amino-2-methyl-1-propanol gebildet ist.

6. Zusammensetzung nach einem der Ansprüche 1 bis 5, dadurch gekennzeichnet, daß der Silikongummi aus der Gruppe ausgewählt ist, die von den Polyalkylsiloxanen mit Trimethylsilyl- oder Trihydrosilylendgruppen und den folgenden Kopolymeren gebildet ist: Poly(dimethylsiloxan/methyl-vinylsiloxan), Poly(dimethylsiloxan/-diphenylsiloxan), Poly(dimethylsiloxan/phenylmethylsiloxan) und Poly(dimethylsiloxan/diphenylsiloxan/methylvinylsiloxan).

7. Zusammensetzung nach einem der Ansprüche 1 bis 6, dadurch gekennzeichnet, daß die Gummen in einem Silikonöl in Mischung oder in Lösung sind.

8. Zusammensetzung nach Anspruch 7, dadurch gekennzeichnet, daß das Silikonöl ein Polydimethylsiloxanöl oder ein Polyphenylmethylsiloxanöl ist.

9. Zusammensetzung nach einem der Ansprüche 1 bis 7, dadurch gekennzeichnet, daß der Silikongummi oder die Mischung von Silikongummi und Silikonöl in einem Lösungsmittel in Lösung ist.

10. Zusammensetzung nach Anspruch 9, dadurch gekennzeichnet, daß das Lösungsmittel aus der Gruppe ausgewählt ist, die von den Isoparaffinen, Methylchlorid, Pentan, Dodekan, Tridekan, Tetradekan und ihren Mischungen gebildet ist.

11. Zusammensetzung nach einem der Ansprüche 1 bis 10, dadurch gekennzeichnet, daß die wässrige Phase der Emulsion 45 bis 90 Gew.-%, bezogen auf das Gesamtgewicht der Zusammensetzung, darstellt, wobei die Ölphase die Ergänzung darstellt.

12. Zusammensetzung nach einem der Ansprüche 1 bis 11, dadurch gekennzeichnet, daß das Kopolymer (C) in der wässrigen Phase der Emulsion in einem Anteil von 0,05 bis 1 Gew.-%, bezogen auf das Gesamtgewicht der Zusammensetzung, und vorzugsweise von 0,1 bis 0,5 Gew.-%,bezogen auf das Gesamtgewicht der Zusammensetzung, vorliegt.

13. Zusammensetzung nach einem der Ansprüche 1 bis 12, dadurch gekennzeichnet, daß die Ölphase zusätzlich zu dem oder den Silikongummen oder der Mischung aus Silikongummi bzw. Silikongummen und Silikonöl mindestens ein Öl enthält, das aus der von den pflanzlichen, tierischen, mineralischen und synthetischen Ölen gebildeten Gruppe genommen ist.

14. Zusammensetzung nach einem der Ansprüche 1 bis 13, dadurch gekennzeichnet, daß die wässrige Phase zusätzlich zu dem Kopolymer (C) mindestens ein vernetztes oder nicht vernetztes Akrylsäure-Homo- oder Kopolymer oder eine seiner Salze enthält.

15. Zusammensetzung nach Anspruch 14, dadurch gekennzeichnet, daß das Akrylsäurepolymer, das nicht das Kopolymer (C) ist, in der wässrigen Phase der Emulsion in einem Anteil von 0,1 bis 2 Gew.-%, bezogen auf das Gesamtgewicht der Zusammensetzung, vorliegt.

16. Kosmetische Verwendung einer Zusammensetzung nach einem der Ansprüche 1 bis 15 als Creme oder Milch für die Hautpflege.

17. Verwendung einer Zusammensetzung nach einem der Ansprüche 1 bis 15 zur Bildung eines Schminkprodukts aus der Gruppe der Make-ups, Wimperntuschen, Wangenschminken, Lidschatten und Eye-Liners.
